# EUROPEAN PATENT SPECIFICATION

(11) **EP 3 962 352 B1**
(45) Date of publication and mention of the grant of the patent: **19.11.2025**
(21) Application number: 20728194.0
(22) Date of filing: 30.04.2020
(51) Int. Cl.: A61B 5/00, A61B 18/00, A61B 5/287

(54) **MONOPHASIC-ENABLED CATHETER WITH MICROELECTRODES**
MONOPHASENAKTIVIERTER KATHETER MIT MIKROELEKTRODEN
CATHÉTER MONOPHASIQUE AVEC MICROÉLECTRODES

(30) Priority: 02.05.2019 US 201962842439 P; 22.04.2020 US 202016855904
(43) Date of publication of application: 09.03.2022
(73) Proprietor: Biosense Webster (Israel) Ltd., Yokneam 2066717 (IL)
(72) Inventor: DATTA, Keshava, Irvine, California 92618 (US); RAO, Anand R., Irvine, California 92618 (US); PENDEKANTI, Rajesh, Irvine, California 92618 (US); FUIMAONO, Kristine, Irvine, California 92618 (US)
(74) Representative: Carpmaels & Ransford LLP
(86) International application number: PCT/IB2020/054062
(87) International publication number: WO 2020/222150

(56) References cited:
- EP-A1- 2 420 196
- EP-A1- 3 292 833
- WO-A1-2008/118992
- WO-A1-2011/014602
- WO-A1-2013/166391
- WO-A1-2014/031865
- WO-A1-2019/023280
- CN-A- 103 584 853
- US-A- 4 979 510
- US-A1- 2012 232 374
- US-A1- 2017 106 461

## Description

### FIELD OF INVENTION

The present description relates generally to electrophysiology catheters, and in particular, irrigated ablation catheters.

### BACKGROUND OF INVENTION

Electrical activity at a point in the heart is typically measured by advancing a multiple-electrode catheter to measure electrical activity at multiple points in the heart chamber simultaneously. A record derived from time varying electrical potentials as measured by one or more electrodes is known as an electrogram. Electrograms may be measured by unipolar or bipolar leads, and are used, e.g., to determine onset of electrical propagation at a point, known as local activation time. Various electrode designs are known for different purposes. In particular, catheters having basket-shaped electrode arrays are known and described, for example, in U.S. Pat. No. 5,772,590.

An electrogram is bi-phasic as well as being a global signal. Thus, sensors in a cardiac chamber may detect far-field electrical activity, i.e., the ambient electrical activity originating away from the sensors, which can distort or obscure local electrical activity, i.e., signals originating at or near the sensor location. Thus, in some instances, it is desirable to obtain a local signal in the form of a monophasic action potential signal. Monophasic action potentials (MAPs) are extracellularly recorded wave forms that can reproduce the repolarization time course of transmembrane action potentials (TAPs) with high fidelity. Applicants recognized that there is a need to provide a catheter that can obtain a local signal in the form of a MAP signal.

EP3292833A1 discusses an apparatus that includes a catheter and a tip electrode, at a distal end of the catheter, shaped to define a plurality of microelectrode apertures. The apparatus further includes at least one printed circuit board (PCB) disposed within a lumen of the catheter, and a plurality of microelectrodes coupled to the PCB and at least partly situated within the microelectrode apertures, the PCB being configured to carry signals from the microelectrodes.

WO2013/166391A1 discusses an irrigated ablation catheter adapted for direct tissue contact that has micro-elements that provide more accurate sensing of tissue, including thermal and electrical properties for temperature and impedance and intracardiac ECG measurements. A pressure sensing assembly adds the ability to measure the force at the tip of the catheter as well as to have the micro-elements for accurately sensing tissue parameters. A system uses signals from the micro-elements (impedance, temperature, and ECG signals) as well as the measure of force or pressure at the tip electrode order to provide the operator with a means to control lesion depth, size, transmurality and to ablate tissue until successful treatment of an arrhythmia is achieved. US 2017/106461 A1 discloses a catheter with micro-electrodes protruding therefrom.

### SUMMARY OF THE DISCLOSURE

MAP has been used in electrophysiology to allow for a better understanding at a cellular level of the tissue response. The MAP can reproduce the repolarization time course of transmembrane action potentials (TAPs) with high fidelity with the use of an active electrode and an inactive electrode. Embodiments of the present invention include a catheter with microelectrodes and thermocouples so that the microelectrodes can be utilized to cause a localized therapeutic trauma on the tissue to study MAP on the local tissue.

Embodiments of the present invention obtain MAP signals by using an aspiration catheter with a sensing catheter to create a localized trauma in tissue which causes a response in measurable signals from the tissue. The MAP signal is used to show effects of drugs, diseased or healthy tissues, among other diagnosticable indicators. Embodiments of the present invention also obtain MAP signals by using a catheter to apply pressure on the tissues to obtain reversible localized injury on the tissue. Either of these techniques allows a health care provider to infer the cellular level response (i.e., signals) due to a local trauma so that a therapeutic response can be devised.

Embodiments of the present invention include a catheter with multi-microelectrodes with thermocouples to obtain MAP signals by using contact force-applying microelectrodes to provide an optimum force on the tissue (for a reversible localized injury) while measuring the response signals from the tissue with the force-applying microelectrodes. The MAP signals can be measured as well as with the non-force-applying microelectrodes.

The microelectrodes allow for consistent force application due to a contact force sensor via the smaller surface area in which the microelectrodes are applied against, along with a roughened or fractured surface that allow for extraction of high signal to noise electrical signals from the localized tissue injury.

The invention is defined by claim 1 with additional embodiments defined by the dependent claims.

### BRIEF DESCRIPTION OF THE DRAWINGS

These and other features and advantages of the present invention will be better understood by reference to the following detailed description when considered in conjunction with the accompanying drawings wherein:
FIG. 1 is a schematic, pictorial illustration of a catheter ablating system, according to an embodiment;
FIG. 2 is side perspective view of a distal section of a monophasic-enabled catheter with multiple microelectrodes suitable for use with the system of FIG. 1, according to an embodiment.
FIG. 3 is a side cross-sectional view of the distal section of FIG. 2.
FIG. 4A are pre-ablation ECGs by microelectrodes detecting MAP signals.
FIG. 4B are 3-D electroanatomical maps and post-ablation ECGs by the microelectrodes in the absence of MAP signals following successful ablation.
FIG. 4C are post-ablation ECGs by the microelectrodes following movement of the microelectrodes to a new tissue target location.
FIG. 5 is a side cross-sectional view of the distal section of FIG. 2, with sensing portions of the microelectrodes generally buried in tissue with sufficient force to create reversible localized injury for detecting ECG signals with MAP characteristics.

### DETAILED DESCRIPTION OF EMBODIMENTS

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%. In addition, as used herein, the terms "patient," "host," "user," and "subject" refer to any human or animal subject and are not intended to limit the systems or methods to human use, although use of the subject invention in a human patient represents a preferred embodiment.

### OVERVIEW

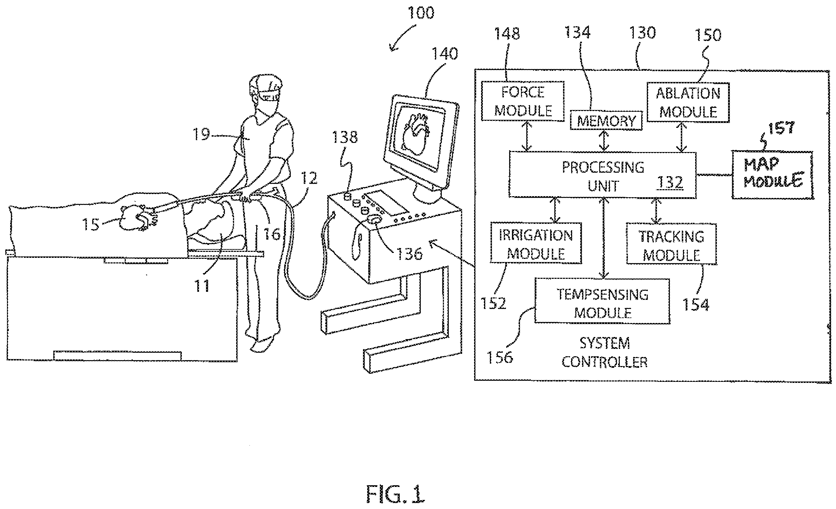

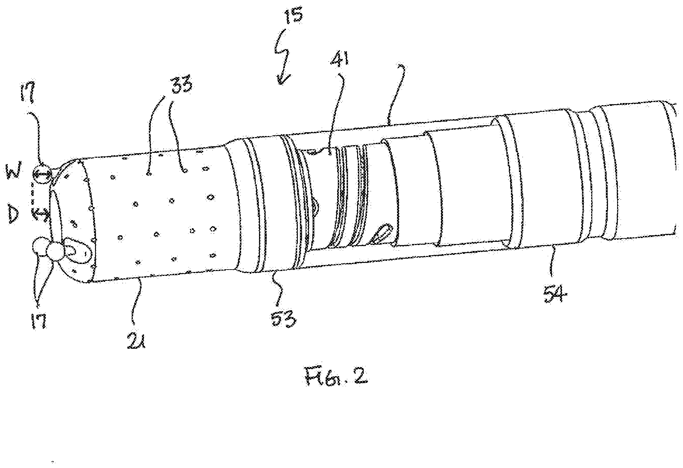

With reference to **FIG. 1** and **FIG. 2****,** a catheter 10, which can be used in a minimally invasive procedure such as ablation of cardiac tissue, comprises an elongated catheter shaft 12 and a shorter deflection section 14 distal of the catheter shaft 12, which can be deflected unidirectionally or bi-directionally. Suitable embodiments of the catheter shaft 12 and deflection section 14 are described in U.S. Application Serial No. Application No. 15/925,521, filed March 19, 2018, and titled CATHETER WITH MULTIFUNCTIONAL MICROINJECTION-MOLDED HOUSING. Distal of the deflection section 14 is a distal section 15 which includes a force sensor 40 and a tip electrode 21 supporting a plurality of microelectrodes 17 and a plurality of thermocouples 18. The catheter also includes a control handle 16 proximal of the catheter shaft 12.

### SYSTEM DESCRIPTION

As shown in FIG. 1, which is a schematic, pictorial illustration of a catheter ablation system 100. In system 100, the catheter 10 is inserted into the vascular system of patient 11 and into a chamber of a heart 13. The catheter is used by an operator 19 of system 100, during a procedure which typically includes performing ablation of the patient's heart tissue.

The operations, functions and acts of system 100 are managed by a system controller 130, comprising a processing unit 132 communicating with a memory 134, wherein is stored software for operation of system 100. In some embodiments, the controller 130 is an industry-standard personal computer comprising a general-purpose computer processing unit. However, in some embodiments, at least some of the operations, functions or acts of the controller are performed using custom-designed hardware and software, such as an application specific integrated circuit (ASIC) or a field programmable gate array (FPGA). In some embodiments, the controller 130 is managed by the operator 19 using a pointing device 136 and a graphic user interface (GUI) 138, which enable the operator to set parameters of system 100. The GUI 138 typically also displays results of the procedure to the operator on a display monitor 140.

The software in memory 134 may be downloaded to the controller in electronic form, over a network, for example. Alternatively or additionally, the software may be provided on non-transitory tangible media, such as optical, magnetic, or electronic storage media.

Electrical components, including electrodes, thermocouples and position (location or orientation) sensors, of the distal section 15 are connected to system controller 130 by conductors that pass through the catheter shaft 12 and the deflection section 14. In addition to being used for ablation, the electrodes may perform other functions, as is known in the art. The system controller 130 may differentiate between the currents for the different functions of the electrical components by frequency multiplexing. For example, radio-frequency (RF) ablation power may be provided at frequencies of the order of hundreds of kHz, while position sensing frequencies may be at frequencies of the order of 1 kHz. A method of evaluating the position of distal section 15 using impedances measured with respect to the electrodes is disclosed in U.S. Patent No. 8,456,182 titled "Current Localization Tracker," to Bar-Tal et al.

As shown in **FIG. 1****,** the system controller 130 includes a force module 148, an RF ablation module 150, an irrigation module 152, a tracking module 154, a temperature sensing module 156 and a MAP module 157. The system control 130 uses the force module 148 to generate and measure signals supplied to, and received from, a force sensor 40 in the distal section 15 in order to measure the magnitude and direction of the force on distal section 15. The system controller 130 uses the ablation module 150 to monitor and control ablation parameters such as the level of ablation power applied via the one or more electrodes of the distal section 15. The ablation module 150 includes an RF generator (not shown) and controls the power/wattage and duration of ablation being applied.

Typically, during ablation, heat is generated in the one or more electrodes energized by the ablation module 150, as well as in the surrounding region. In order to dissipate the heat and to improve the efficiency of the ablation process, the system controller 130 monitors temperature of different portions/surfaces of the distal section 15 and supplies irrigation fluid to distal section 15. The system controller 130 uses the irrigation module 152 to monitor and control irrigation parameters, such as the rate of flow and the temperature of the irrigation fluid. In some embodiments, the system controller 130 uses the irrigation module 152 in response to the temperature sensing module 156 in managing "hot spots" or uneven heating on the surface of the distal section 15, by controlling and adjusting movable internal components of the distal section 15, as described in detail further below.

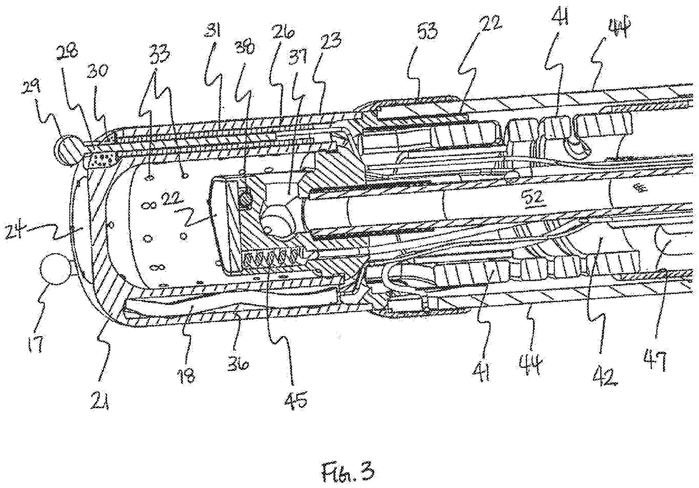

The system controller 130 uses the tracking module 154 to monitor the location and orientation of the distal section 15 relative to the patient 11. The monitoring may be implemented by any tracking method known in the art, such as one provided in the Carto3^{®} system manufactured by Biosense Webster of Irvine, CA. Such a system uses radio-frequency (RF) magnetic transmitter external to patient 11 and responsive elements (e.g., a position sensor 50, see) within distal section 15. Alternatively or additionally, the tracking may be implemented by measuring impedances between **FIG. 3** one or more electrodes, and patch electrodes attached to the skin of patient 11, such as is also provided in the Carto3^{®} system. For simplicity, elements specific to tracking and that are used by module 154, such as the elements and patch electrodes referred to above, are not shown in **FIG. 1****.**

The system controller 130 uses the MAP module 157 to receive and process MAP signals sensed by the microelectrodes in reproducing repolarization time course of transmembrane action potentials (TAPs) with high fidelity with the use of an active electrode and an inactive electrode. As described in detail further below, the MAP signals pre- and post-ablation can provide an indication to an operator of the system as to where and when to move the catheter to create a continuous lesion or line of block.

With reference to **FIG. 2****,** and **FIG. 3****,** the distal section 15 includes a shell cap electrode 21 configured with a proximal neck 22, a cylindrical side wall 23 and a distal end 24 that surround an internal chamber 25 having a proximal opening at the neck 22 that is configured to receive an insert 20 that occupies the proximal opening. The cap electrode 21 is configured for one or more functions, including, for example, ablation. The side wall 23 includes multiple radial irrigation apertures 33 that allow fluid inside the chamber 25 to exit to outside the cap electrode 21. The side wall 23 also includes a plurality of longitudinal through-bores 26 positioned in equi-angular locations about a center longitudinal axis 27 of the distal section 15. In the illustrated embodiment, three bores 26 are located at about 0, 120 and 240 degrees about the axis 27, although it is understood that the plurality of bores may differ, for example, between 2 and 5, as needed or desired. Each bore 26 has a proximal opening and a distal opening, and each bore 26 extends the length of the side wall between the neck 22 and the distal end 24 of the cap electrode 21. The cap electrode 21 may be constructed of any suitable material, including, for example, platinum palladium.

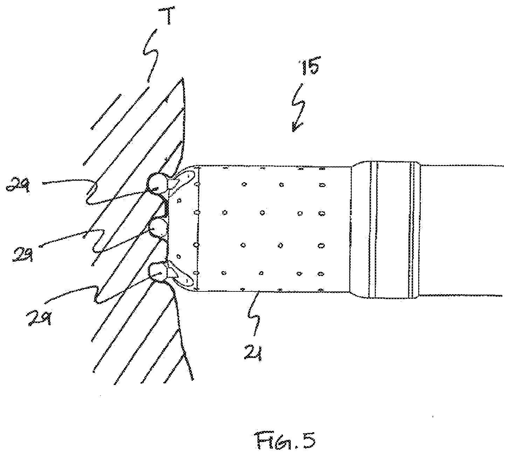

Extending within each bore 26 is a respective microelectrode 17 having an elongated stem 28 and a distal sensing portion 29 that is exposed and configured for contact with tissue. The microelectrode 17 may be constructed of any material, including, for example, platinum iridium. Notably, the stem 28 of each microelectrode 17 is configured to extend a predetermined distance distal of the distal end 24 of the cap electrode 21 so that the distal sensing portion 29 can contact and indent the tissue T with optimum force to cause a reversible localized trauma, but without causing permanent injury, for sensing MAP signals, as shown in **FIG. 5****.** The distal sensing portions 29 of the microelectrodes 17 have their contact surface protruding above the surface topology of the electrode 21 so the distal sensing portions 29 can be generally buried in the tissue. At each distal end of a bore 26, a recess 30 is formed the distal end 24 of the cap electrode 21. The recess 30 may be filled with a material, e.g., polyurethane, to seal and pot the distal portion of the stem 28 in the recess 30.

The distal sensing portion 29 of the microelectrode 17 is configured, having a spherical or bulbous configuration that can be generally fully enveloped by surrounding tissue so as to avoid sensing extracellular or far-field signals. The profile of the microelectrodes serves to cause reversible perforation for studying MAPs at the tissue site. With multiple microelectrodes, multiple separate local tissue area can be studied simultaneously. The configuration of the distal sensing portion may include oval or elliptical configurations. In some embodiments, the distal portion 29 has a width or diameter W of about 0.014 mm and 0.015 mm and the stem 28 has a length of about 0.100 mm. The protrusion distance D of distal sensing portion 29 measured from a distalmost surface of the distal sensing portion 29 to a distal face of the distal end 24 is about 0.023 mm. The protrusion distance enables the microelectrodes access to in depth MAPs of the localized cellular tissue.

In some embodiments, the surface of the distal sensing portion 29 are mechanically prepared so as to minimize signal noise via cleaning methodologies and surface coatings. In some embodiments, a surface of the distal sensing portion 29 is roughened, for example, by plasma etching, or coated with one or more coatings of fracturing substance, for example, silver chloride, iridium oxide or titanium nitride, to provide cracks and crevices on the order of microns to increase the surface area of the distal portion. Iridium oxide can provide up to 100 times greater surface area. Titanium nitride can provide up to 1000 times greater surface area. Mechanical roughening with plasma etching can provide up to 10 times greater surface area. Such fractured surface area allows for extraction of high signal to noise electrical signals from the localized tissue trauma.

Each stem 28 is surrounded by an elongated insulating support member 31 with a lumen 32, for example, a polyimide tube, that is generally coextensive with the stem in the respective bore 26. The member 31 electrically isolates the entirety of the microelectrode 17 from the electrode 21. The fit between the stem 28 and the lumen 32, and the fit between the support member 31 and the bore 26 may be a close or tight fit. A distal end of the insulating support member 31 is configured with a flange 34 to seal the bore 26 and the lumen 32. At a proximal end of stem 28, electrical connection is provided, for example, by welding, to a respective lead wire 35. The proximal opening of each bore 26 leads into the neck 22 of the cap electrode 21 so that the lead wires 35 can extend into the neck 22 and proximally along the deflection section 14 and the catheter shaft 12 toward the control handle 16.

The side wall 23 of the cap electrode 21 also has a plurality of blind passages 36 in equi-angular locations about the center longitudinal axis 27, offset from the locations of the bores 26, each housing a respective thermocouple (TC) wire pair 18 for example a constantan wire and a copper wire pair. In some embodiment, six blind passages 36 are located in the side wall to house six pairs of TC 18, for example, at 15, 75, 135, 195, 255 and 315 degrees about the axis 27. Twisted distal ends of a wire pair forming a distal junction of each TC 18 are housed in a respective tube 39, for example, a hypotube, that has a predetermined length greater than the length of the blind passages. The greater length of the hypotubes and the distal junctions, and a larger diameter of the blind passages 36 enable the hypotubes and the distal junctions to be crammed into a nonlinear shape inside the blind passages so that contact between the hypotubes and the inner wall of blind passages is ensured for more accurate temperature sensing of the cap electrode 21. Proximal opening of each blind passage opens into the neck 22 of the cap electrode 21 so that the wire pairs of the TC 18 can pass into the neck and proximally along the catheter deflection section 14, the catheter shaft 12 and into the control handle 16.

In some embodiments, the insert 20 is configured in part as an irrigation fluid flow diverter with one or more radial channels 37 that provide fluid communication between the chamber 25 and a distal end of an irrigation lumen 52 that extends along the length of the catheter between the distal section 15 and the control handle 16. The irrigation module 152 of the system controller 130 (**FIG. 1**) controls the flow of irrigation fluid through the irrigation lumen 52 and into the chamber 25.

The insert 20 occupying the neck 22 of the cap electrode 21 may be formed with a blind hole to receive a distal end of lead wire 55 for energizing the insert 20 and the cap electrode 21. A transverse channel may also be formed through which a safety wire 38 passes to tether the cap electrode 21 to the catheter 10 as a safety measure. In some embodiments, the distal section 15 includes a force sensor 40 whose distal end is connected to the proximal end of the insert. Aspects of a similar force sensor are described in U.S. Patent No. 8,357,152, to Govari et al., issued January 22, 2013, and in U.S. Patent Application 2011/0130648, to Beeckler et al., filed November 30, 2009. The force sensor 40 comprises a resilient coupling member 41, which forms a spring joint between distal and proximal ends of the coupling member, with a central lumen 42 therethrough. The coupling member 41 typically has one or more helices 43 cut in the member 41, so that the member 41 behaves as a spring.

The coupling member 41 is mounted within and covered by a nonconducting, biocompatible sheath 44, which is typically formed from flexible plastic material. Having the outer diameter of the coupling member to be as large as possible, typically increases the sensitivity of force sensor 40. In addition, and as explained below, the relatively large diameter of the tubular coupling member 41, and its relatively thin walls, provide the relatively spacious central lumen 42 through which components pass into and out of the distal section 15. During RF ablation procedures, considerable heat may be generated in the distal section 15 and thus the sheath 44 may comprise a heat-resistant plastic material, such as polyurethane, whose shape and elasticity are not substantially affected by exposure to the heat.

In some embodiments, the force sensor 40 includes a distal coil 45 (**FIG. 3**) housed in the insert 20 distal of the spring joint, and three proximal coils 46 (not shown) proximal of the spring joint. The coils provide accurate reading of any dimensional change in the spring joint of the force sensor 40, including axial displacement and angular deflection of the joint. These coils are one type of magnetic transducer that may be used in embodiments of the present invention. A "magnetic transducer," in the context of the present patent application and in the claims, means a device that generates a magnetic field in response to an applied electrical current or outputs an electrical signal in response to an applied magnetic field. Although the embodiments described herein use coils as magnetic transducers, other types of magnetic transducers may be used in alternative embodiments, as will be apparent to those skilled in the art.

In some embodiments, the distal coil 45 is driven by a current, via a cable (not shown) from the system controller 130 and the force module 148, to generate a magnetic field. This field is received by the proximal coils 46 which are fixed at the same axial distance from the coil 45 but at different angular locations about the longitudinal axis 27, for example, 0, 120, and 240 degrees about the axis 27. Proximal coils 46 generate electrical signals in response to the magnetic field transmitted by the distal coil 45. These signals are conveyed by a cable (not shown) to the system controller 130, which uses the force module 148 to process the signals in order to measure the displacement of spring joint parallel and concentric with axis 27, as well as to measure the angular deflection of the joint from the axis. From the measured displacement and deflection, the system controller 130 is able to evaluate, typically using a previously determined calibration table stored in force module 148, a magnitude and a direction of the force on the spring joint of the coupling member 41. Notably, the force sensor 40 enables the plurality of microelectrodes 17 to apply a consistent force against the tissue, although it is understood that the catheter 10 in some embodiments need not have a force sensor.

The system controller 130 uses the tracking module 154 (**FIG. 1**) to measure and detect the location and orientation of distal end 12. The method of detection may be by any convenient process known in the art. In some embodiments, magnetic fields generated external to patient 11 (e.g., by generators positioned below patient's bed) generate electric signals in a position sensor 50 housed in the lumen 42 of the coupling member 41 generally proximal of the spring joint. As understood by one of ordinary skill in the art, the position sensor 50 comprises sensing coil X, coil Y, and coil Z (which in some embodiments is one of the coils 46). The system controller 130 processes the electric signal to evaluate the location and orientation of the distal section 15. Alternatively, the magnetic fields may be generated in the distal section 15, and the electrical signals created by the fields may be measured external to patient 11.

In use, the catheter 10 is introduced into the patient's vascular system and the distal section 15 is advanced to an area of interest, for example, a heart chamber. The system controller 130 accomplishes diagnostic procedures, including mapping. For example, the position sensor 50 generates signals processed by the tracking module 154 in determining location and orientation of the distal section 15. The tip electrode 21, a distal ring electrode 53 and/or a proximal ring electrode 54 sense electrical activity of heart tissue which signals generated are processed by processing unit 132. A 3-D electrophysiology map may be created from these processed signals, and ablation tissue sites are identified and targeted. The system controller 130 may then accomplish therapeutic procedures. For example, the operator maneuvers the distal section 15 so that the tip electrode 21 is in contact with the targeted tissue site. Contact between the tip electrode 21 and tissue results in the application of a force that displaces the distal section 15 relative to the proximal end of the coupling member 41 of the force sensor 40. Such displacement causes the proximal coils 46 to generate signals that are processed by the force module 148, for example, to confirm contact of the distal section 15 and tissue in preparation for ablation.

Before and/or during ablation, the irrigation module 152 controls delivery and rate of delivery of irrigation fluid to the distal section 15 by a pump (not shown) that delivers irrigation fluid from a fluid source (not shown) through the irrigation lumen 52. The ablation module 150 delivers RF energy to the cap electrode 21 which heats the target tissue to form a lesion. One or more of the thermocouples TCs 18 generate signals representative of temperature of respective surrounding tissue and fluids. Depending on the temperature(s) sensed, the system controller 130 may in some embodiments communicate with the ablation module 150 to adjust the power delivery and/or with the irrigation module 152 to adjust the rate of fluid delivery or the position of the flow director 58 to its distal-most position, a more distal position or a less proximal position, as appropriate to avoid hot-spots, charring or thrombosis. Irrigation fluid can therefore be directed to exit the irrigation apertures 33 at one or more selected flow rates.

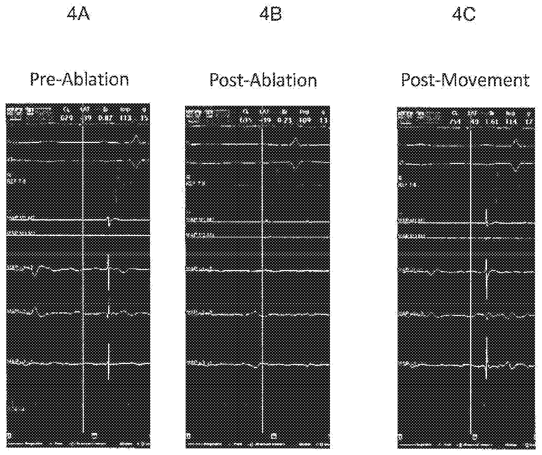

By pressing one or more microelectrodes 17 against tissue with sufficient force to bury the respective one or more distal sensing portions 29 into the tissue to cause reversible localized trauma or injury, the one or more microelectrodes 17 can detect MAP signals. Three pre-ablation ECG signals detected respectively by the microelectrodes 17, designated µ1-µ2, µ2-µ3, and µ3-µ1, as shown in **FIG. 4A****,** exhibit monophasic characteristics that are distinctive from biphasic ECG signals detected by the other electrodes. In contrast, the three post-ablation ECG signals detected by the same microelectrodes, as shown in **FIG. 4B****,** exhibit no monophasic activity - these signals are generally flatline, whereas other electrodes continue to sense signals. With ablation procedure forming effective lesions, the microelectrodes detect no MAP signals indicating that the target tissue has been successfully necrosed. In **FIG. 4C****,** the distal section 15 of the catheter has been moved so that the same microelectrodes are embedded in new target location; hence, the ECG signals detected by the microelectrodes again exhibit monophasic characteristics. Thus, in some embodiments, a method (not falling within the scope of the claims) of ablating using the aforementioned catheter with microelectrodes includes:
- positioning catheter with one or more microelectrodes in tissue contact at a first location along a desired ablation pattern;
- acquiring pre-ablation ECG signals as sensed by the one or more microelectrodes at the first location, the ECG signals having monophasic action potential characteristics;
- performing ablation with the catheter at the first location;
- acquiring post-ablation ECG signals as sensed by the microelectrodes at the first location; and
- repositioning the catheter with the one or more microelectrodes in tissue contact to a second location along the desired ablation pattern solely when the post-ablation ECG signals at the first location are devoid of the monophasic action potential characteristics.

The method may also include:
- reperforming ablation at the first location when at least a portion of the monophasic characters remains present in the post-ablation ECG signals.

The above method may be particularly useful when a continuous lesion or line of block is desired, such as for pulmonary vein isolation.

## Claims

1. A catheter (10) comprising:
an elongated catheter shaft (12);
a distal section (15), including;
an ablation electrode (21) having a side wall (23) and an outer surface, the side wall having (23) a plurality of bores (26);
a plurality of microelectrodes (17), each microelectrode having:
a distal sensing portion (29) that protrudes from the outer surface of the ablation electrode (21), wherein the distal sensing portion (29) has a bulbous configuration, and
an elongated stem (28) extending through a respective one of the plurality of bores (26) of the ablation electrode (21);
wherein
the plurality of microelectrodes are configured to sense monophasic action potential signals;
**characterized in that**
the distal section (15) further includes a force sensor (40) configured to sense contact force of the plurality of microelectrodes (17) against a tissue surface; and
the elongated stem (28) of each microelectrode extends a predetermined distance distal of a distal end of the ablation electrode (21).

2. The catheter of claim 1, wherein the distal sensing portion has a fractured surface.

3. The catheter of claim 1, wherein the distal sensing portion has a coating from the group consisting of silver chloride, iridium oxide and titanium oxide.

4. The catheter of claim 1, wherein the distal sensing portion has an etched surface.

5. The catheter of claim 1, wherein the distal sensing portion has a width ranging between about 0.014 mm and 0.015 mm.

6. The catheter of claim 1, wherein the distal sensing portion is configured to cause reversible localized injury to tissue.

7. The catheter of claim 1, wherein the side wall of the ablation electrode includes at least one blind passage (36) and at least one thermocouple wire pair (18) in the blind passage.

8. The catheter of claim 7, wherein the thermocouple wire pair (18) is housed in a hypotube (39) that has a predetermined length greater than the length of the blind passage (36) and inserted into the blind passage such that the wire pair has a nonlinear shape so as to provide at least one contact surface with an interior surface of the blind passage.

## Patentansprüche

1. Katheter (10), umfassend:
einen länglichen Katheterschaft (12);
einen distalen Abschnitt (15), der einschließt;
eine Ablationselektrode (21) mit einer Seitenwand (23) und einer Außenoberfläche, wobei die Seitenwand (23) eine Vielzahl von Bohrungen (26) aufweist;
eine Vielzahl von Mikroelektroden (17), wobei jede Mikroelektrode aufweist:
einen distalen Sensorabschnitt (29), der aus der Außenoberfläche der Ablationselektrode (21) hervorsteht, wobei der distale Sensorabschnitt (29) eine bauchige Gestalt aufweist, und
einen länglichen Schaft (28), der sich durch jeweils eine der Vielzahl von Bohrungen (26) der Ablationselektrode (21) erstreckt;
wobei die Vielzahl von Mikroelektroden konfiguriert ist, um monophasische Aktionspotentialsignale zu erfassen;
**dadurch gekennzeichnet, dass**
der distale Abschnitt (15) ferner einen Kraftsensor (40) einschließt, der konfiguriert ist, um die Kontaktkraft der Vielzahl von Mikroelektroden (17) gegen eine Gewebeoberfläche zu erfassen; und
der verlängerte Schaft (28) jeder Mikroelektrode sich über eine vorbestimmte Distanz distal eines distalen Endes der Ablationselektrode (21) erstreckt.

2. Katheter nach Anspruch 1, wobei der distale Sensorabschnitt eine gebrochene Oberfläche aufweist.

3. Katheter nach Anspruch 1, wobei der distale Sensorabschnitt eine Beschichtung aus der Gruppe bestehend aus Silberchlorid, Indiumoxid und Titanoxid aufweist.

4. Katheter nach Anspruch 1, wobei der distale Sensorabschnitt eine geätzte Oberfläche aufweist.

5. Katheter nach Anspruch 1, wobei der distale Sensorabschnitt eine Breite im Bereich zwischen etwa 0,014 mm und 0,015 mm aufweist.

6. Katheter nach Anspruch 1, wobei der distale Sensorabschnitt konfiguriert ist, um eine reversible lokal begrenzte Gewebeverletzung zu verursachen.

7. Katheter nach Anspruch 1, wobei die Seitenwand der Ablationselektrode mindestens einen Blindkanal (36) und mindestens ein Thermoelement-Leiterpaar (18) im Blindkanal einschließt.

8. Katheter nach Anspruch 7, wobei das Thermoelement-Leiterpaar (18) in einem Hyporohr (39) untergebracht ist, das eine vorbestimmte Länge aufweist, die größer ist als die Länge des Blindkanals (36), und so in den Blindkanal eingeführt ist, dass das Leiterpaar eine nichtlineare Form aufweist, um mindestens eine Kontaktfläche mit einer Innenoberfläche des Blindkanals bereitzustellen.

## Revendications

1. Cathéter (10) comprenant :
un corps de cathéter allongé (12) ;
une section distale (15), comportant ;
une électrode d'ablation (21) ayant une paroi latérale (23) et une surface extérieure, la paroi latérale ayant (23) une pluralité de trous (26) ;
une pluralité de microélectrodes (17), chaque microélectrode ayant :
une partie de détection distale (29) qui dépasse de la surface extérieure de l'électrode d'ablation (21), dans lequel la partie de détection distale (29) a une configuration bulbeuse, et
une tige allongée (28) s'étendant à travers l'un de la pluralité d'alésages (26) de l'électrode d'ablation (21) ;
dans lequel la pluralité de microélectrodes est configurée pour détecter des signaux de potentiel d'action monophasiques ;
**caractérisé en ce que**
la section distale (15) comporte en outre un capteur de force (40) configuré pour détecter la force de contact de la pluralité de microélectrodes (17) contre la surface d'un tissu ; et
la tige allongée (28) de chaque microélectrode s'étend à une distance distale prédéterminée de l'extrémité distale de l'électrode d'ablation (21).

2. Cathéter selon la revendication 1, dans lequel la partie sensorielle distale présente une surface fracturée.

3. Cathéter selon la revendication 1, dans lequel la partie sensorielle distale est recouverte d'un revêtement du groupe constitué par le chlorure d'argent, l'oxyde d'indium et l'oxyde de titane.

4. Cathéter selon la revendication 1, dans lequel la partie sensorielle distale présente une surface gravée.

5. Cathéter selon la revendication 1, dans lequel la portion de détection distale a une largeur comprise entre environ 0,014 mm et 0,015 mm.

6. Cathéter selon la revendication 1, dans lequel la partie sensorielle distale est configurée pour provoquer une lésion localisée réversible du tissu.

7. Cathéter selon la revendication 1, dans lequel la paroi latérale de l'électrode d'ablation comporte au moins un passage aveugle (36) et au moins une paire de fils de thermocouple (18) dans le passage aveugle.

8. Cathéter selon la revendication 7, dans lequel la paire de fils de thermocouple (18) est logée dans un hypotube (39) qui a une longueur prédéterminée supérieure à la longueur du passage aveugle (36) et inséré dans le passage aveugle de telle sorte que la paire de fils a une forme non linéaire afin de fournir au moins une surface de contact avec une surface intérieure du passage aveugle.
